# EUROPÄISCHE PATENTSCHRIFT

(11) **EP 0 517 000 B1**
(45) Veröffentlichungstag und Bekanntmachung des Hinweises auf die Patenterteilung: **11.10.1995**
(21) Anmeldenummer: 92107846.5
(22) Anmeldetag: 08.05.1992
(51) Int. Cl.: F16K 7/06

(54) **Schlauchklemme für medizinische Zwecke**
Hose clamp for medical use
Pince pour tuyaux souples pour usage médical

(30) Priorität: 07.06.1991 DE 4118732
(43) Veröffentlichungstag der Anmeldung: 09.12.1992
(73) Patentinhaber: JOKA KATHETERTECHNIK GMBH, D-72379 Hechingen (DE)
(72) Erfinder: Funk, Gerald, W-7457 Bisingen 3-Wessingen (DE); Hartig, Tomas, W-7450 Hechingen (DE)
(74) Vertreter: Möbus, Rudolf, Dipl.-Ing.

(56) Entgegenhaltungen:
- DE-A- 3 003 527
- DE-U- 8 812 121
- FR-A- 2 440 512
- US-A- 1 580 649
- US-A- 3 822 052
- US-A- 4 053 135
- US-A- 4 097 020

## Beschreibung

Die Erfindung betrifft eine Schlauchklemme für medizinische Zwecke mit einem bügelartigen an den Bügelenden zu einem geschlossenen Ring rastverbindbaren Klemmenkörper, der Öffnungen für einen in der Bügelebene verlaufenden Durchgangskanal für den abzuklemmenden Schlauch und an beiden Bügelschenkeln je eine nach innen gerichtete, keilförmige Klemmbacke aufweist. Schlauchklemmen der genannten Art werden von der Anmelderin bereits vertrieben. Sie haben seitlich offene Klemmenkörper, so daß der Schlauch an der Klemmstelle von außen sichtbar bleibt. Eine solche Schlauchklemme ist zum Beispiel in der US Patentschrift 4,097,020 gezeigt. Dabei besteht aber die Gefahr daß sich ein sehr weicher und entsprechend flexibler Schlauch aus dem Klemmenkörper seitlich aus der Bügelebene ausbiegt mit der Folge, daß beim Schließen der Schlauchklemme die Klemmbacken den Schlauch nicht mehr oder nur noch teilweise erfassen.

Diese Gefahr ist besonders groß, wenn die Schlauchklemme an Krümmungsstellen eines Schlauches angesetzt werden muß.

Der Erfindung liegt die Aufgabe zugrunde, eine Schlauchklemme der eingangs genannten Art so auszubilden, daß mit ihr auch in Krümmungsbereichen eines Schlauches ein sicheres Erfassen des Schlauches durch die Klemmbacken gewährleistet ist.

Die gestellte Aufgabe wird mit der eingangs genannten Schlauchklemme erfindungsgemäß dadurch gelöst, daß die Schlauchklemme zu beiden Seiten des Durchgangskanales parallel zu der Bügelebene gerichtete Seitenwandungen aufweist, die in der Schlauchklemme ein Auswölben des Schlauches aus der Bügelebene verhindern. Vorteilhafterweise können die Seitenwandungen dabei so bemessen sein, daß sie beide Klemmbacken mindestens teilweise seitlich abdecken.

Durch die Seitenwandungen wird der Schlauch in unmittelbarer Nahe der Klemmbacken mit Sicherheit daran gehindert, aus der Ebene des seitlich offenen Klemmenkörpers auszubrechen Die Seitenwandungen können an den Klemmenkörper angeformt sein. Bei einer bevorzugten Ausführungsform sind die Seitenwandungen an einem gesonderten Einsatzkörper für den Klemmenkörper ausgebildet. Dies hat den Vorteil, daß kompliziertere Spritzgußformen für die aus Kunststoff hergestellten Klemmenkörper vermieden werden und daß die Schlauchklemmen wahlweise mit oder ohne die Einsatzkörper eingesetzt werden können. Bei relativ steifen Schläuchen kann nämlich auf die Seitenwandungen wie bisher verzichtet werden, und der Einsatzkörper kann auswechselbar im Klemmenkörper angeordnet sein.

Der ebenfalls mit einfachen Spritzgußformen herstellbare Einsatzkörper kann vorteilhafterweise U-förmig sein, wobei die U-Schenkel die Seitenwandungen bilden, und in der U-Basis kann eine Schlauchdurchgangsöffnung mit parallel zu den Seitenwandungen gerichteter Öffnungsachse ausgebildet sein. Zweckmäßig kann die U-Basis des Einsatskörpers dicker als die Seitenwandungen ausgebildet sein, deren gegenseitiger freier Abstand mindestens der Dicke des Klemmenkörpers an der Einsatzstelle entspricht. Durch die Seitenwandungen wird die Dicke des Klemmenkörpers nur geringfügig erhöht, und die Seitenwandungen bilden glatte Abschlußflächen, die bei einem Aufliegen der Schlauchklemme auf der Haut eines Patienten keine unangenehmen Druck- und Scheuerstellen auf der Haut bilden können. Der Einsatzkörper läßt sich durch einfaches Aufweiten der Bügelschenkel in den Raum zwischen den beiden miteinander zusammenwirkenden Klemmbacken und dem Verbindungsbogen der Bügelschenkel einklipsen und zum Auswechseln auch wieder ausklipsen. In seiner Einsatzlage fluchtet die Schlauchdurchgangsöffnung des Einsatzkörpers mit einer Durchgangskanalöffnung für den abzuklemmenden Schlauch in dem Verbindungsbogen des Klemmenkörpers.

Nachfolgend wird ein Ausführungsbeispiel einer bevorzugten Ausführungsform der Schlauchklemme anhand der beigefügten Zeichnung näher erläutert.

Im einzelnen zeigen:
- Fig. 1: einen zentralen Längsschnitt durch den Klemmenkörper der Schlauchklemme;
- Fig. 2: eine Seitenansicht der Schlauchklemme mit in den Klemmenkörper eingeklipstem Einsatzkörper;
- Fig. 3: einen Schnitt durch die Schlauchklemme entlang der Linie III-III in Fig. 2;
- Fig. 4: eine Einzeldarstellung des Einsatzkörpers der Schlauchklemme in einer Aufsicht A, einer Seitenansicht B und einer stirnseitigen Ansicht C.

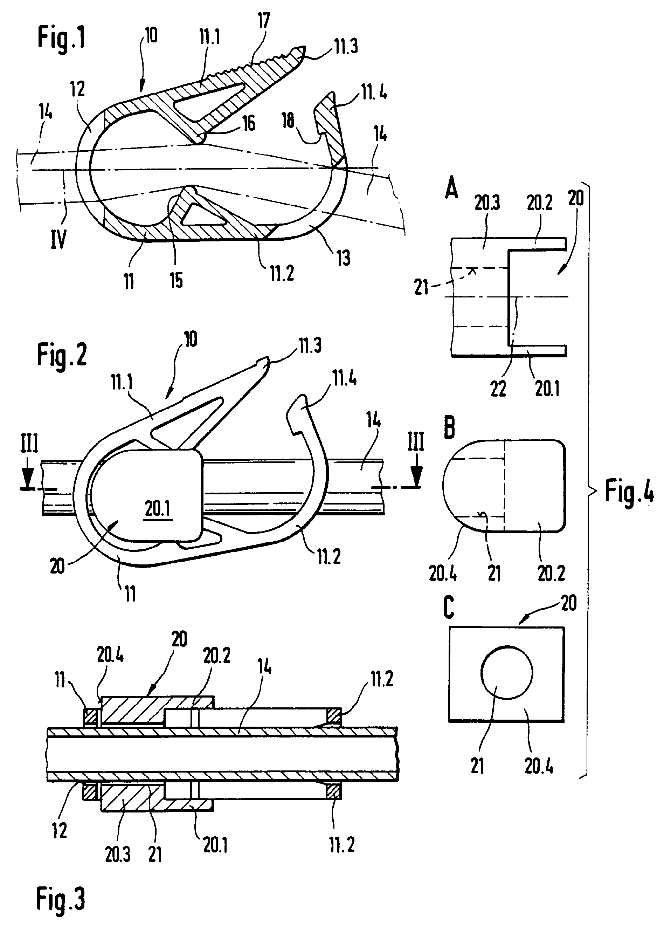

Fig. 1 zeigt einen zentralen Längsschnitt durch den bügelartigen Klemmenkörper 11 der Schlauchklemme 10, der bereits Stand der Technik ist. Der Klemmenkörper 11 weist eine erste Durchgangsöffnung 12 und eine zweite Durchgangsöffnung 13 für einen abzuklemmenden Schlauch 14 auf, der in Fig. 1 mit strichpunktierten Linien angedeutet ist. An den beiden Bügelschenkeln 11.1 und 11.2 des Klemmenkörpers 11 ist jeweils eine keilartige Klemmbacke 15 oder 16 angeformt, die gegeneinanderbewegt werden und den Schlauch 14 abklemmen, wenn die Enden 11.3 und 11.4 der beiden Bügelschenkel 11.1 und 11.2 zum Schließen des bügelförmigen Klemmenkörpers 11 gegeneinanderbewegt werden. Das Ende 11.3 des Bügelschenkels 11.1 ist als Rastzunge ausgebildet. An dem Ende 11.4 des anderen Bügelschenkels 11.2 ist innenseitig eine Raststufe 18 ausgebildet, in welche das Ende 11.3 des anderen Bügelschenkels 11.1 einrasten kann. Das Schließen der Bügelschenkel zu einem geschlossenen ringförmigen Klemmenkörper 11 erfolgt in bekannter Weise durch Fingerdruck auf eine Außenriffelfläche 17 des Bügelschenkels 11.1. Das Öffnen der Schlauchklemme 10 erfolgt durch Fingerdruck in Auswärtsrichtung auf das Ende 11.4 des anderen Bügelschenkels 11.2.

Fig. 2 zeigt die Schlauchklemme 10 in Seitenansicht mit einem eingeklipsten Einsatzkörper 20. Der Einsatzkörper 20 ist in Fig. 4 einzeln in drei Ansichten dargestellt. Aus Fig. 4 und der Schnittdarstellung der Fig. 3 ist ersichtlich, daß der Einsatzkörper 20 U-förmig ausgebildet ist und seine beiden U-Schenkel dünne Seitenwandungen 20.1 und 20.2 bilden, welche den seitlich offenen Klemmenkörper 11 von seinem die Durchgangsöffnung 12 tragenden Verbindungsbereich zwischen den beiden Bügelschenkeln 11.1 und 11.2 bis zu den beiden Klemmbacken 15 und 16 und noch etwas darüber hinaus abdecken. Die U-Basis 20.3 des Einsatzkörpers 20 ist wesentlich stärker als die beiden Seitenwandungen 20.1 und 20.2 ausgebildet und mit einer Durchgangsöffnung 21 versehen, deren Längsachse parallel zu den beiden Seitenwandungen 20.1, 20.2 verläuft. Das rückseitige Ende 20.4 des Einsatzkörpers 20 ist abgerundet und damit an den Verbindungsbogen des Klemmenkörpers 11 im Bereich der Durchgangsöffnung 12 angepaßt. Durch Aufweiten der beiden Bügelschenkel 11.1 und 11.2 läßt sich der Einsatzkörper 20 zwischen den beiden Klemmbacken 15 und 16 hindurch in den Klemmenkörper 11 einklipsen.

## Patentansprüche

1. Schlauchklemme für medizinische Zwecke, mit einem bügelartigen, an den Bügelenden zu einem geschlossenen Ring rastverbindbaren Klemmenkörper (11), der Öffnungen (12, 13) für einen in der Bügelebene verlaufenden Durchgangskanal für den abzuklemmenden Schlauch (14) und an beiden Bügelschenkeln (11.1, 11.2) je eine nach innen gerichtete, keilförmige Klemmbacke (15, 16) aufweist, dadurch gekennzeichnet, daß sie zu beiden Seiten des Durchgangskanales parallel zu der Bügelebene gerichtete Seitenwandungen (20.1, 20.2) aufweist, die in der Schlauchklemme (10) ein Auswölben des Schlauches (14) aus der Bügelebene verhindern.

2. Schlauchklemme nach Anspruch 1, dadurch gekennzeichnet, daß die Seitenwandungen (20.1, 20.2) beide Klemmbacken (15, 16) mindestens teilweise seitlich abdecken.

3. Schlauchklemme nach Anspruch 1 oder 2, dadurch gekennzeichnet, daß die Seitenwandungen (20.1, 20.2) an einem gesonderten Einsatzkörper (20) für den Klemmenkörper (11) ausgebildet sind.

4. Schlauchklemme nach Anspruch 3, dadurch gekennzeichnet, daß der Einsatzkörper (20) auswechselbar im Klemmenkörper (11) angeordnet ist.

5. Schlauchklemme nach Anspruch 3 oder 4, dadurch gekennzeichnet, daß der Einsatzkörper (20) U-förmig ist, wobei die U-Schenkel die Seitenwandungen (20.1, 20.2) bilden und in der U-Basis (20.3) eine Schlauchdurchgangsöffnung (21) mit parallel zu den Seitenwandungen (20.1, 20.2) gerichteter Öffnungsachse (22) ausgebildet ist.

6. Schlauchklemme nach einem der Ansprüche 3 bis 5, dadurch gekennzeichnet, daß die U-Basis (20.3) des Einsatzkörpers (20) dicker als die Seitenwandungen (20.1, 20.2) ausgebildet ist, deren gegenseitiger freier Abstand mindestens der Dicke des Klemmenkörpers (11) an der Einsatzstelle entspricht.

7. Schlauchklemme nach einem der Ansprüche 3 bis 6, dadurch gekennzeichnet, daß der Einsatzkörper (20) durch Aufweiten der Bügelschenkel (11.1, 11.2) des Klemmenkörpers (11) in den Raum zwischen den beiden miteinander zusammenwirkenden Klemmbacken (15, 16) und dem Verbindungsbogen der Bügelschenkel (11.1, 11.2) einklipsbar ist, wo seine Schlauchdurchgangsöffnung (21) mit einer Durchgangskanalöffnung (12) für den abzuklemmenden Schlauch (14) in dem Verbindungsbogen des Klemmenkörpers (11) fluchtet.

## Claims

1. Hose clamp for medical use, with a stirrup-like clamp body (11) which can be connected lockably at the stirrup ends to form a closed ring and which has openings (12, 13) for a channel passage, extending in the plane of the stirrup, for the hose (14) which is to be clamped, and which has, on both stirrup legs (11.1, 11.2), in each case an inwardly directed, wedge-shaped clamp jaw (15, 16), characterized in that it has, on both sides of the channel passage, side walls (20.1, 20.2) which are directed parallel to the plane of the stirrup and which prevent the hose (14) in the hose clamp (10) from bulging out from the plane of the stirrup.

2. Hose clamp according to Claim 1, characterized in that the side walls (20.1, 20.2) laterally cover both clamp jaws (15, 16) at least partially.

3. Hose clamp according to Claim 1 or 2, characterized in that the side walls (20.1, 20.2) are formed on a separate insert body (20) for the clamp body (11).

4. Hose clamp according to Claim 3, characterized in that the insert body (20) is arranged exchangeably in the clamp body (11).

5. Hose clamp according to Claim 3 or 4, characterized in that the insert body (20) is U-shaped, the legs of the U forming the side walls (20.1, 20.2), and a hose passage opening (21) with an opening axis (22) directed parallel to the side walls (20.1, 20.2) being formed in the U base (20.3).

6. Hose clamp according to one of Claims 3 to 5, characterized in that the U base (20.3) of the insert body (20) is designed thicker than the side walls (20.1, 20.2), the free space between the latter corresponding at least to the thickness of the clamp body (11) at the insert position.

7. Hose clamp according to one of Claims 3 to 6, characterized in that, by spreading apart the stirrup legs (11.1, 11.2) of the clamp body (11), the insert body (20) can be clipped into the space between the two interacting clamp jaws (15, 16) and the connecting arch of the stirrup legs (11.1, 11.2), where its hose passage opening (21) is aligned with a channel passage opening (12), for the hose (14) to be clamped, in the connecting arch of the clamp body (11).

## Revendications

1. Pince pour tuyaux souples pour usage médical, avec un corps de pince (11) en forme d'étrier, pouvant être relié par crans par les extrémités d'étrier en un anneau fermé (11) qui comprend des ouvertures (12, 13) pour un canal de passage pour le tuyau souple à pincer (14) développé dans le plan de l'étrier et à chacune des deux branches d'étrier (11.1, 11.2) une mâchoire de serrage en forme de coin (15, 16) orientée vers l'intérieur, caractérisée en ce que des deux côtés du canal de passage elle comprend des parois latérales (20.1, 20.2) orientées parallèlement au plan d'étrier qui empêchent dans la pince pour tuyaux souples (10) un bombement du tuyau souple (14) hors du plan d'étrier.

2. Pince pour tuyaux souples selon la revendication 1, caractérisée en ce que les parois latérales (20.1, 20.2) recouvrent latéralement au moins partiellement les deux mâchoires de serrage (15, 16).

3. Pince pour tuyaux souples selon la revendication 1 ou 2, caractérisée en ce que les parois latérales (20.1, 20.2) sont formées sur un corps d'insert séparé (20) pour le corps de pince (11).

4. Pince pour tuyaux souples selon la revendication 3, caractérisée en ce que le corps d'insert (20) est monté interchangeable dans le corps de pince (11).

5. Pince pour tuyaux souples selon la revendication 3 ou 4, caractérisée en ce que le corps d'insert (20) est en forme de U, les branches du U formant les parois latérales (20.1, 20.2) et une ouverture de passage de tuyau souple (21) avec un axe d'ouverture (22) orienté parallèlement aux parois latérales (20.1, 20.2) étant formée dans la base du U (20.3).

6. Pince pour tuyaux souples selon l'une des revendications 3 à 5, caractérisée en ce que la base du U (20.3) du corps d'insert (20) est formée plus épaisse que les parois latérales (20.1, 20.2), dont l'écartement libre réciproque correspond au moins à l'épaisseur du corps de pince (11) au point d'insertion.

7. Pince pour tuyaux souples selon l'une des revendications 3 à 6, caractérisée en ce que le corps d'insert (20) peut être enclipsé par écartement des branches d'étrier (11.1, 11.2) du corps de pince (11) dans l'espace entre les deux mâchoires de serrage (15, 16) coopérant l'une avec l'autre et l'arc de liaison des branches d'étrier (11.1, 11.2), à l'endroit où son ouverture de passage de tuyau souple (21) s'aligne avec une ouverture de canal de passage (12) pour le tuyau souple à pincer (14) dans l'arc de liaison du corps de pince (11).
